(19) Europäisches Patentamt
European Patent Office
Office européen des brevets

(11) Publication number: **0 193 944**
**A1**

# (12) EUROPEAN PATENT APPLICATION

(21) Application number: **86102903.1**

(22) Date of filing: **05.03.86**

(51) Int. Cl.[4]: **C 07 D 239/54**
// A61K31/505

(30) Priority: **07.03.85 JP 43584/85**

(43) Date of publication of application: **10.09.86**
**Bulletin 86/37**

(84) Designated Contracting States: **DE FR GB IT**

(71) Applicant: **Nippon Kayaku Kabushiki Kaisha, 11-2, Fujimi-cho 1 chome Chiyoda-ku, Tokyo (JP)**

(72) Inventor: **Izawa, Takao, 1090, Kamiochiai, Yono-shi Saitama-ken (JP)**
Inventor: **Kato, Kuniki, 5-24-4, Oto, Yono-shi Saitama-ken (JP)**
Inventor: **Suzuki, Masanobu, 1090, Kamiochiai, Yono-shi Saitama-ken (JP)**
Inventor: **Nishikawa, Kiyohiro, 3-17-1-402, Shimo Kita-ku, Tokyo (JP)**
Inventor: **Nagahata, Takemitsu, 1068-10, Yachigasaki, Matsudo-shi Chiba-ken (JP)**

(74) Representative: **Türk, Dietmar, Dr. rer. nat. et al, Patentanwälte Türk & Gille Brucknerstrasse 20, D-4000 Düsseldorf 13 (DE)**

(54) **Derivatives of 5-fluorouracil.**

(57) This invention relates an antitumor-active 5-fluorouracil derivative represented by the general formula

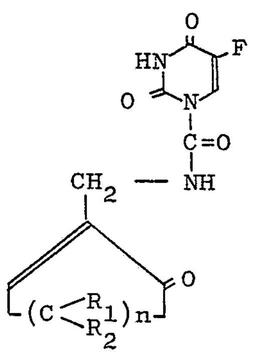

wherein $R_1$ and $R_2$ represent hydrogen atom or lower alkyl group, and n is zero or an integer of 1, and salts thereof.

EP 0 193 944 A1

# Derivatives of 5-Fluorouracil

## Background of the Invention

5-Fluorouracil (hereinafter referred to as "5-FU") (see USP 2,885,396) has been widely used as a potent antitumor in the clinical field. It has, however, a defect of difficulty of oral administration because of its toxicity to digestive organs.

Therefore, those compounds which are chemically modified 5-FU have been vigorously studied to reduce toxicity along with maintained favorable activity so as to enable oral administration. For example, 1-(2-tetrahydrofuryl)-5-fluorouracil [trade name; Futraful® (Taiho Yakuhin)](see Japanese Patent Publication No. 10510/74) etc. are their representatives.

Since malignant tumor shows various properties and resistant cell against antitumor may also come into existence, a novel antitumor-active substance is continually demanded to make possible oral administration. The compounds according to the present invention are so different from Futraful® in chemical structure. Therefore that are expected as novel successful antitumor.

## Summary of the Invention

As a result of various studies by the inventors about antitumor-active substances, it was found that the 5-FU

derivative represented by the following general formula (I)

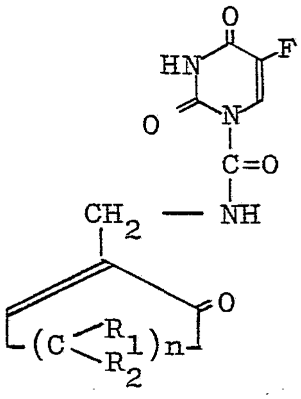

(I)

wherein, $R_1$ and $R_2$ represent hydrogen atom or lower alkyl group respectively, and n is zero or integer of 1, exhibit a excellent antitumor activity even by oral administration also. The present invention has been accomplished based on the above finding.

The above-mentioned lower alkyl groups are, for example, alkyl ($C_{1-3}$) groups such as methyl, ethyl, propyl, isopropyl, etc.

## Detailed Description of the Invention

The representative compounds of the present invention are shown as follows:

1. 1-(4,4-dimethyl-6-oxo-1-cyclohexenyl)methylcarbamoyl-5-fluorouracil
2. 1-(6-oxo-1-cyclohexenyl)methylcarbamyl-5-fluorouracil
3. 1-(5-oxo-1-cyclopentenyl)methylcarbamoyl-5-fluorouracil

A method for producing the compounds according to the present invention represented by the general formula (I) is described below.

A compound of the formula (I) of the present invention can be easily produced from 5-FU represented by the following general formula (II) and the compound represented by the general formula (III):

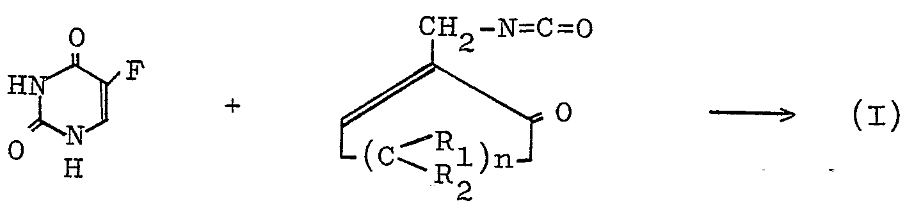

+ → (I)

(II) (III)

, wherein $R_1$, $R_2$ and n represent the same as the above-mentioned,

Namely, the compound (I) according to the present invention can be obtained by reacting 5-FU (II) with an equivalent mol of the compound (III). In practice, 5-FU (II) may be used in excess compared to the amount of the compound (III), in more detail, preferably in 2 to 4 equivalents for reaction. This reaction is carried out

in a polar solvent such as acetone, dimethylformamide, dimethylacetamide, dimethylsulfoxide, etc., particularly in a solvent sufficiently dehydrated by a conventional dehydrating agents, and can be completed about from room temperature to 100°C for several hours or days.

Moreover, this process is an addition reaction, so may be carried usually under neutral conditions, but yield of the reaction may be sometimes increased by adding a catalytic quantity of a base, in particular, organic bases such as triethylamine, pyridine, etc. The said compound may be purified by conventional method, for example, silica-gel column chromatography, and when the target substance crystallizes, it may be recrystalized by a suitable solvent.

The compounds of the present invention have very potent antitumor activity.

The antitumor activity of the compound of the present invention was investigated in the following way:

<u>Experimental Example</u>

Antitumor effect to murine leukemia P388.

Test compounds: 5-FU and compound No.1 (1-(4,4-dimethyl-6-oxo-1-cyclohexenyl)methylcarbamoyl-5-fluorouracil)

Method : 1 x $10^6$ leukemia P 388 cells per mouse were implanted subcutaneously to female

mice ($CDF_1$-SLC strain, 18-21 g of weight). The control and test group consisted of 3 and 1 mice respectively. The compound No.1 and 5-FU were dissolved in 100% dimethylsulfoxide respectively to be adjusted to various concentrations. The solution was administered orally to each mouse once daily for 5 consecutive days, starting 24 hours after the implantation.

Method of evaluation and its results

Average period in days of survival in the group receiving the compound of this invention (T) and average period in days of survival in the control group (C) were determined to calculate survival rates (T/C x 100%). The results are shown in Table below.

| Compound | Dose (mg/kg/inj) | Survival Rate (T/C x 100) % |
|---|---|---|
| 5-FU | 84 | 52.5 |
| | 42 | 64.7 |
| | 21 | 115 |
| | 10.5 | 103 |
| | 5.3 | 103 |
| | 2.7 | 101 |
| Compound No.1 | 400 | 71.9 |
| | 200 | 122 |
| | 100 | 151 |
| | 50 | 129 |
| | 25 | 115 |

As is evident from the results, the survival rate of the compound of the present invention is as dominant so that the compound of this invention is expected to be an antitumor.

In addition, as compared with 5-FU concerning the effective dose, 5-FU shows 10 to 20 mg/kg/inj optimum dose, and in intrast, the compound according to the present invention (compound No.1) does not only permit up to 50 to 200 mg/kg of dose, but extends also the range of dose. These facts indicate that the compound of the present

invention is less toxic than 5-FU and is, moreover, adjustable of dose, so that it is very safe pharmaceutical.

The present invention will be described in detail with reference to the following Examples:

Example 1.

Preparation of 1-(4,4-dimethyl-6-oxo-1-cyclohexenyl) methylcarbamoyl-5-fluorouracil.

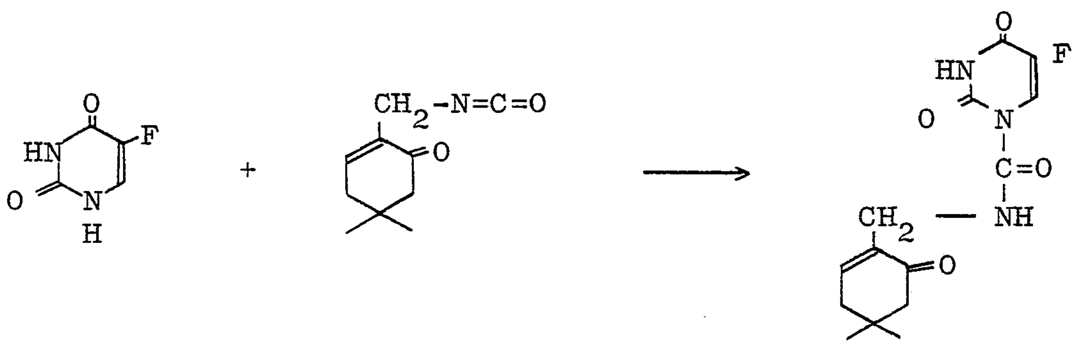

160 mg of 5-FU was heated with 330 mg of (4,4-dimethyl-6-oxo-1-cyclohexenyl)methylisocyanate in 20 ml of anhydrous acetone for 6 days under reflux to cause reaction. The reaction mixture was cooled to room temperature, and then concentrated under reduced pressure. The obtained residue was washed with a small amount of petroleum ether, and the retained raw powder was suspended in 50 ml of chloroform, followed by heating for about 10 minutes under reflux. Soon after that, the hot chloroform solution was filtered through a glass filter, and the obtained filtrate was concentrated under reduced pressure. The white powder

thus obtained was subjected to silica-gel column chromatography with the solution mixture of chloroform/methanol (10:1) as eluent, to obtain 249 mg of the target compound in 65% yield.

m.p. : 191 to 197°C (sublimed)

IR spectrum $\nu_{max.}^{KBr}$ ($cm^{-1}$) : 3330, 3196, 3080, 1756, 1722, 1698, 1673

NMR spectrum $\delta$ ($CDCl_3$) : 1.04 (6H, S, 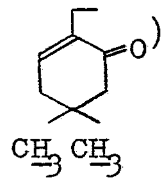)

2.31 (2H, d, J=4.4Hz, 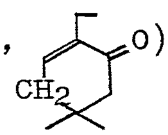)

2.32 (2H, S, 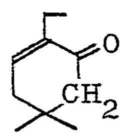)

4.14 (2H, dd, J=5.9, 1.1Hz, 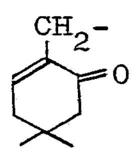)

6.87 (1H, d.d, J=4.4, 1.1Hz, 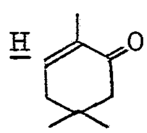)

8.43 (1H, d, J=6.6Hz, 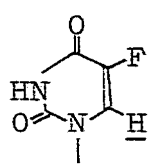)

8.53 (1H, bs, 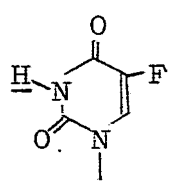)

9.30 (1H, t, J=5.9Hz, 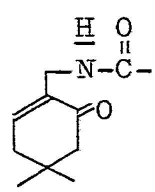)

Mass spectrum (M/Z) : 309 ($C_{14}H_{16}N_3O_4F$)

Example 2.

Preparation of 1-(6-oxo-1-cyclohexenyl)methylcarbamoyl-5-fluorouracil.

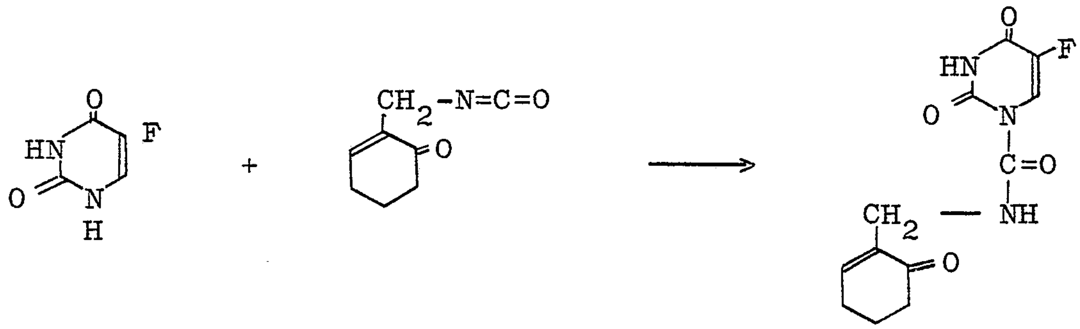

1.5 equivalents of 5FU were reacted with 350 mg of (6-oxo-1-cyclohexenyl)methylisocyanate in a manner similar to Example 1. The resulting reaction mixture was treated with hot chloroform, and then was subjected to silica-gel column chromatography with a solution mixture of chloroform/methanol (10:1) as an eluent to obtain 404 mg of the target compound in 62% yield.

m.p. : 180 - 200°C (wet)
280°C above (decomposed)

IR spectrum $\nu_{max}^{KBr}(cm^{-1})$ : 3330, 3090, 1764, 1720, 1705, 1676, 1664.

NMR spectrum $\delta(CDCl_3)$ : 2.02 (2H, t.t, J=6.0, 6.7Hz, 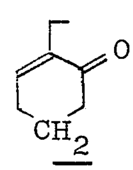

2.42 (2H, d.t, J=4.3, 6.0Hz, 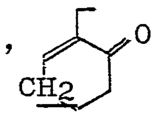)

2.47 (2H, t, J=6.7Hz, 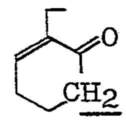)

4.12 (2H, d, d, J=1.1, 6.3Hz, 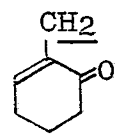)

7.01 (1H, t, t, J=1.1, 4.3Hz,
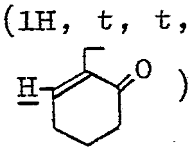 )

8.29 (1H, bs, 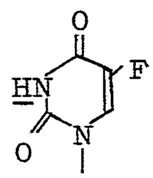)

8.43 (1H, d, J=6.7Hz, 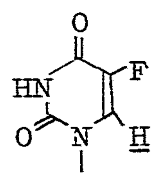)

9.31 (1H, t, J=6.3Hz,
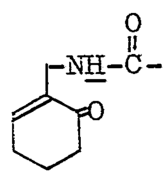 )

Mass spectrum (M/Z) : 281 ($C_{12}H_{12}N_3O_4F$)

<u>Example 3.</u>

Preparation of 1-(5-oxo-1-cyclopentenyl)methylcarbamoyl-5-fluorouracil.

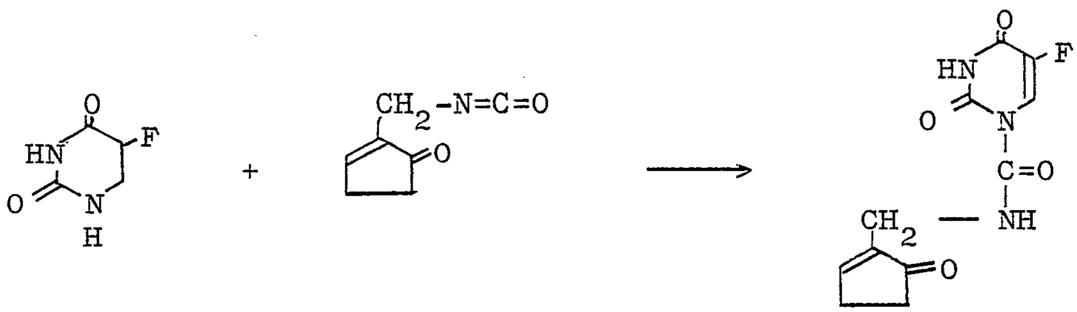

1.5 equivalents of 5FU were reacted with 430 mg of (5-oxo-1-cyclopentenyl)methylisocyanate in a manner and procedure described in Examples 1 and 2, and isolation and purification of reaction product were followed by a similar manner to the preceding Examples to obtain 47.7 mg of the target compound in 57% yield.

m.p. : 175 - 185°C (wet)
240° above (decomposed)

IR spectrum $\nu_{max}^{KBr}(cm^{-1})$ : 3290, 3092, 1748, 1730, 1706, 1682

NMR spectrum $\delta(CDCl_3)$ : 2.47 (2H, m, 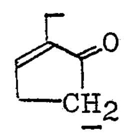 )

2.56 (2H, m, 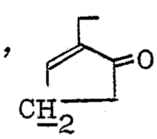 )

4.20 (2H, m, 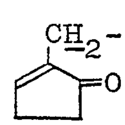 )

7.52 (1H, m, 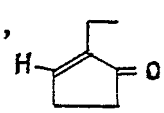 )

8.44 (1H, d, J=6.7Hz, 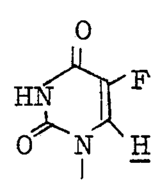 )

8.45 (1H, bs, 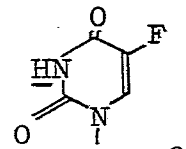 )

9.36 (1H, m, 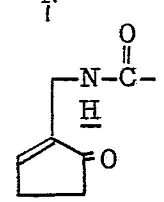 )

Mass spectrum (M/Z) : 267 ($C_{11}H_{10}N_3O_4F$)

Claims

1. A 5-fluoroaracil derivative represented by the general formula

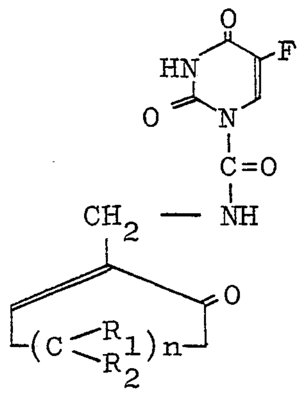

wherein $R_1$ and $R_2$ represent hydrogen atom or lower alkyl group, and n is zero or an integer of 1.

2. The 5-fluorouracil derivative according to Claim 1, wherein $R_1$ and $R_2$ represent hydrogen or methyl group

3. 1-(4,4-dimethyl-6-oxo-1-cyclohexenyl)methylcarbamoyl-5-fluorouracil and salt thereof.

4. 1-(6-oxo-1-cyclohexenyl)methylcarbamoyl-5-fluorouracil.

5. 1-(5-oxo-1-cyclopentenyl)methylcarbamoyl-5-fluorouracil.

European Patent Office

# EUROPEAN SEARCH REPORT

0 193 944

EP 86102903.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A1 - 0 010 941 (FUJISAWA)<br>* Abstract *<br>-- | 1 | C 07 D 239/54<br>//A 61 K 32/505 |
| A | GB - A - 1 524 640 (MITSUI)<br>* Claim 1 *<br>---- | 1 | |
| | | | TECHNICAL FIELDS SEARCHED (Int. Cl.4) |
| | | | C 07 D 239/00 |

The present search report has been drawn up for all claims

| Place of search | Date of completion of the search | Examiner |
|---|---|---|
| VIENNA | 10-06-1986 | LUX |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82